Europäisches Patentamt

European Patent Office

Office européen des brevets

(19)

(11) Publication number: **0 098 341**
**B1**

(12) **EUROPEAN PATENT SPECIFICATION**

(45) Date of publication of patent specification: **16.04.86**

(51) Int. Cl.[4]: **C 07 C 17/38, C 07 C 19/08**

(21) Application number: **83100145.8**

(22) Date of filing: **10.01.83**

(54) Anhydrous phase separation for recovering hydrogen fluoride from a fluorination reaction.

(30) Priority: **08.06.82 US 386467**

(43) Date of publication of application:
**18.01.84 Bulletin 84/03**

(45) Publication of the grant of the patent:
**16.04.86 Bulletin 86/16**

(84) Designated Contracting States:
**BE DE FR GB IT**

(56) References cited:
**US-A-2 478 362**
**US-A-2 640 086**
**US-A-4 209 470**

(73) Proprietor: **PENNWALT CORPORATION**
**Pennwalt Building Three Parkway**
**Philadelphia Pennsylvania 19102 (US)**

(72) Inventor: **Pittard, Fred W.**
**3006 Clay Apt. 4**
**Paducah Kentucky 42001 (US)**

(74) Representative: **Kraus, Walter, Dr. et al**
**Patentanwälte Kraus, Weisert & Partner**
**Thomas-Wimmer-Ring 15**
**D-8000 München 22 (DE)**

Courier Press, Leamington Spa, England.

EP 0 098 341 B1

## Description

The invention is directed to a process for separating hydrogen fluoride from the organic constituents of a reaction mixture containing pentafluorobutane.

The fluorination of methyl chloroform or vinylidene chloride to manufacture 1-chloro-1,1-difluoroethane (designated "142b" under American Society of Refrigeration Engineers numbering system for naming fluorocarbons) results in a product stream which includes unreacted starting materials as well as byproducts including HCl, 1,1-dichloro-1-fluoroethane (designated "141b") and 1,1,1-trifluoroethane (designated "143a"). The reactor bottoms include unreacted HF, pentafluorobutane and small amounts of other organics including tars. Most of the unreacted and partially reacted chlorides are stripped from the product stream and returned to the reactor. The HCl is then stripped from the product stream. By chilling the remaining product stream, a phase separation can be achieved in which one phase contains about 85—90 mole% HF and the other phase contains about 85—90 mole% organics, most of which is 1-chloro-1,1-difluoroethane. Additional distillation of either or both phases and recycling to the phase separator is used to further separate the anhydrous HF from the organics. The HF is then returned to the reaction. US—A—4,209,470 describes such a process in which certain auxiliary solvents, 1,1-dichloro-1-fluoroethane, vinylidene chloride, 1,1,1-trichloroethane (methyl chloroform) and mixtures thereof are used in the phase separation. According to disclosure of this patent, the auxiliary solvents reduce the solubility of the HF in the organic phase and provide immediate separation of the product stream into phases which otherwise would take an extremely long time.

US—A—2,478,362 describes a process for separating valuable components from reaction mixtures obtained in the fluorination of perchloro-ethylene. It has been found, however, that fast and satisfactory separation is achieved without the use of such solvents if contamination is avoided and good temperature control is exercised during the separation.

During the fluorination reaction, reactor bottoms or "tars" build up and the production rate slows down to a point where the reaction mixture must be dumped, for example, in the proportion of about one pound of tars to thirteen pounds of product. The typical composition of the tars is about, by weight, 70% HF, 5—15% 142b, 5 to 20% pentafluorobutane and the remainder 141b, miscellaneous high boiling organics, and HCl. These tars have been discarded in the past after neutralization of the acid. This not only presents a disposal problem but results in the loss of a considerable amount of HF and 142b product. I have now found a process for recovering most of the previously discarded HF and product from the reactor tars which is compatible with the remainder of the product recovery process and apparatus.

In accordance with this invention there is provided a process for separating hydrogen fluoride from the organic constituents of a reaction mixture, which comprises withdrawing a product stream which includes HF and 1-chloro-1,1-difluoroethane from the reaction mixture, withdrawing a tar stream from the reaction mixture, heating the tar to vaporize the HF and the volatile organic material including pentafluorobutane from the tar, combining the vaporized HF and volatile organic material including pentafluorobutane from the tar with the product stream, cooling the resulting mixture to a temperature of from about −26.1 to −9.44°C (about −15 to +15°F) so that the mixture separates into a HF-rich liquid phase and a 1-chloro-1,1-difluoroethane-rich liquid phase, and separating the two phases, a good temperature control being exercized during the separation. A product stream, which includes HF and 1-chloro-1,1-difluoroethane, is withdrawn from the reaction mixture. A tar stream is also withdrawn from the reaction mixture and heated to vaporize the HF and volatile organic material. The vaporized HF and volatile organic material are then combined with the product stream and the resulting mixture is cooled to cause a phase separation of the mixture into a HF-rich liquid phase and a 1-chloro-1,1-difluoroethane rich liquid phase. The two phases are then separated. The HF can be returned to the reaction, with or without removing the residual organic materials. The 1-chloro-1,1-difluoroethane-rich phase is further separated to obtain a purified product.

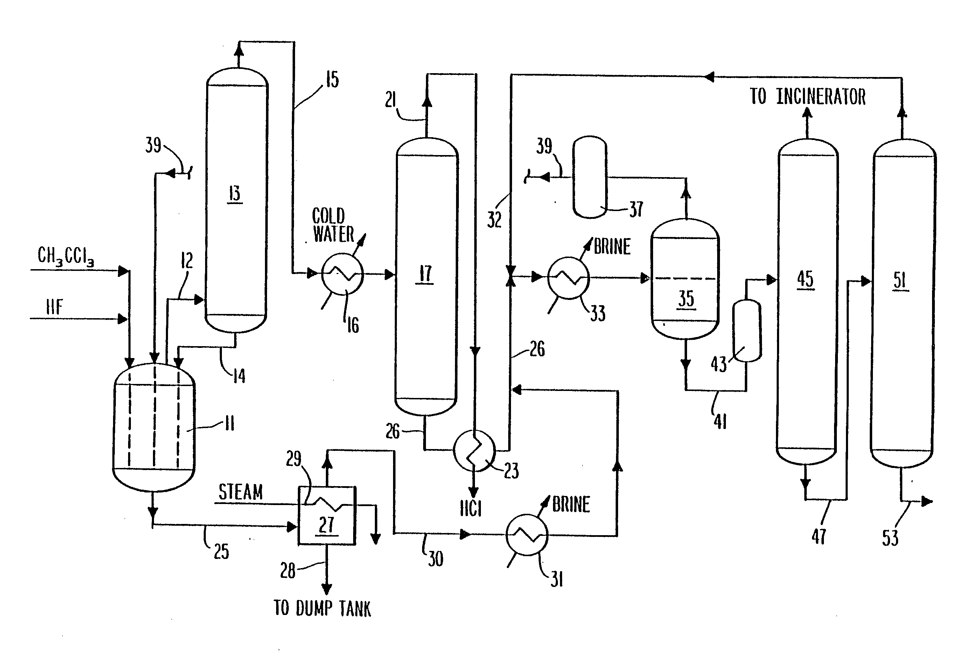

The Figure is a schematic flow diagram illustrating an embodiment of the process of the invention.

A main organic ingredient of the reactor bottoms or "tars" in the fluorination of methyl chloroform is pentafluorobutane. This compound has been found to contribute to the mutual solubility of HF and 1-chloro-1,1-difluoroethane. This indicates that the presence of such a material would hinder a phase separation process. Surprisingly, I have found that this does not occur and the volatile portion of the reactor tars can be combined with the product stream in an anhydrous phase separation process so that the HF in the reactor tars can be recovered at the same time that the HF is separated from the product stream. A minimum amount of additional equipment is needed to accomplish the recovery. A vaporizer is used to separate the actual tar from the reactor bottoms, and a cooler is used prior to combining the volatile material with the product stream. The tar withdrawal can be periodic or continuous so as to maintain a satisfactory reaction rate in the reactor.

Turning now to the Figure, HF and methyl chloroform from supply tanks (not shown) are pumped to reactor 11 where a portion of the methyl chloroform is converted to 1-chloro-1,1-difluoroethane (142b). The crude product gas is withdrawn at the top of reactor 11 and is fed through line 12 to reactor stripper column 13. The bottoms from column 13, which include unreacted methyl chloroform and HF plus some reaction products, are returned through line 14 to reactor 11. This provides a product stream in line 15 containing total amounts of methyl chloroform, vinylidene chloride, and 1,1-di-chloro-1-fluoroethane which are less than 0.01 mole per mole of 142b and keeps the amounts of unreacted or partially reacted materials to a minimum. The bulk of these materials are, therefore, returned to the reactor for conversion into 142b product. The presence of vinylidene chloride in the product stream is undesirable due to its tendency to form explosive peroxides. The presence of these materials is also unnecessary for rapid and efficient phase separation. The reaction products of HF and methyl chloroform from the top of stripper column 13 are cooled and fed through line 15 and cooler 16 to HCl stripper column 17. Stripper column 17 separates an anhydrous stream of HCl in overhead line 21 which contains only trace amounts of HF and organic material. The HCl stream at about −16.1°C (3°F) is used to cool the crude product stream in line 26 to about 55.0°C (131°F) by means of economizer 23. The crude product stream which contains, depending upon the reaction and stripper column conditions, by weight, from about 86 to 89% 142b, from about 9 to 12% HF, and from about 0.5 to 3.5% of 143a, 141b, and other miscellaneous organics along with a trace of HCl, is then flashed through a HCl stripper level control valve (not shown) to a pressure of about 1.35 bar (5 psig) and a temperature of 10.6°C (51°F). A vapor-liquid mixture exits at this point with about 15 weight percent of the stream being vapor.

The reactor bottoms or "tars" are periodically or continuously withdrawn through line 25 to vaporizer 27 which is heated by steam coil 29. The volatile material in the tars represents about 98% by weight of the tar stream, (for example, about 70% HF, 6.0% pentafluorobutane, 14% 142b and 6% 141b and the remainder miscellaneous organics and HCl). The two percent by weight of actual tars or sludge is removed through line 28 to a water containing dump tank for disposal. The gases leaving vaporizer 27, which is operated at about 1.35 bar (5 psig), are at a temperature of about 51.7°C (125°F) when the vaporizer bottom temperature is maintained from about 57.2 to 60.0°C (135° to 140°F). When the bottom temperature reaches 60.0°C (140°F) the sludge is dumped through line 28 into water. The volatile material in line 30 is fed to cooler 31 where it is cooled by refrigerant at a temperature of, for example, −28.9°C (−20°F) prior to being combined with the crude product stream in line 26 and the overhead stream in line 32 from azeotrope column 51. The composition of the azeotrope overhead stream is, by weight, about 86.0 wt.% 142b and 14.0 wt.% HF. The combined streams are condensed and cooled with refrigerant in low temperature heat exchanger 33 to a temperature of from about −26.1 to −9.44°C (−15° to +15°F), and preferably about −20.0°C (−4°F), before entering phase separator 35. The optimum temperature is chosen to give the maximum separation of HF and 142b as determined by solubility curves. For optimum results, good temperature control is maintained. An example of the composition of the total feed to separator 35 is, by weight about 83.8% 1-chloro-1,1-difluoroethane (142b), 13.3% HF, 1.8% 1,1,1-trifluoroethane (143a), 0.6% 1,1-di-chloro-1-fluoroethane (141b), 0.4% pentafluoro-butane and 0.1% HCl.

In the separator, which is operated at a temperature, for example, of about −20.0°C (−4°F) and a pressure of about 1.02 bar (+0.15 psig) the feed stream rapidly separates into an HF-rich upper phase and a 142b-rich lower phase. The HF-rich phase contains, by weight, about 60% HF, 38.6% 142b and the remainder miscellaneous organics and HCl. The HF-rich phase is fed to hold tank 37. The HF-rich phase is pre-heated to 98.9°C (210°F) with steam in a heat exchanger (not shown) before being pumped through line 39 back to reactor 11. The 142b-rich phase from separator 35 contains, by weight, about 94.6% 142b, 2.2% HF, 2.1% 143a, 0.65% 141b, 0.4% pentafluorobutane and 0.1% miscellaneous organics and HCl. The 142b-rich stream in line 41 is pumped from hold tank 43 to light-ends column 45, which is operated, for example, at about 9.0 bar (116 psig) and 32.2°C (90°F) at the top. The purpose of column 45 is to purge the lower-boiling organic components from the system which would otherwise build up in the process. The bottoms from column 45, containing, by weight, about 96.7% 142b, 2.2% HF and 1.1% high boiling organics are fed by line 47 to azeotrope column 51. In azeotrope column 51, which is operated, for example, at 4.4 bar (50 psig) and 27.2°C (81°F) at the top, the 142b/HF azeotrope is removed at the overhead and recycled back to separator 35. The essentially HF free bottoms stream in line 53 containing about 98.7% by weight of 142b product is removed for further purification. The conditions and proportions in the foregoing description are for illustration only and should not be construed as limiting the scope of the invention.

The process of the invention maintains the advantages of an anhydrous recovery of HF and permits the recovery and separation of most of the HF and 1-chloro-1,1-difluoroethane product from the reactor "tars" at the same time. Despite the presence in the "tars" of materials such as pentafluorobutane which would be expected to interfere with the phase separation, a rapid and efficient separation is achieved. The process greatly reduces the quantity of materials which must be disposed of as waste.

**Claims**

1. A process for separating hydrogen fluoride from the organic constituents of a reaction mixture containing pentafluorobutane, comprising withdrawing a product stream which includes HF and 1-chloro-1,1-difluoroethane from the reaction mixture, withdrawing a tar stream from the reaction mixture, heating the tar to vaporize the HF and the volatile organic material including pentafluorobutane from the tar, combining the vaporized HF and volatile organic material including pentafluorobutane from the tar with the product stream, cooling the resulting mixture to a temperature of from about −26.1 to −9.44°C (−15 to +15°F) so that the mixture separates into a HF-rich liquid phase and a 1-chloro-1,1-difluoroethane-rich liquid phase, and separating the two phases, a good temperature control being exercized during the separation.

2. The process of claim 1 wherein the mixture is cooled to a temperature of about −20.0°C (about −4°F).

3. The process of claim 1 including removing unreacted and partially reacted organic material from the product stream and returning said materials to the reaction mixture.

4. The process of claim 1 including removing HCl from the product stream.

5. The process of claim 1 including returning the HF-rich liquid upper phase to the reaction mixture.

6. The process of claim 1 including distilling an azeotropic mixture of HF and 1-chloro-1,1-difluoroethane from the 1-chloro-1,1-difluoroethane-rich liquid phase and combining the azeotropic mixture with the product stream and the HF and volatile organic material from the tar for cooling and phase separation.

7. The process of claim 1 including cooling the HF and volatile organic material from the tar prior to mixing it with the product stream.

**Patentansprüche**

1. Verfahren zur Abtrennung von Fluorwasserstoff von den organischen Bestandteilen eines Reaktionsgemisches, das Pentafluorbutan enthält, dadurch gekennzeichnet, daß ein Produktstrom, der HF and 1-Chlor-1,1-difluoroethan enthält, aus dem Reaktionsgemisch abgezogen wird, ein Terrstrom aus dem Reaktionsgemisch abgezogen wird, der Terr erhitzt wird, um das HF und das flüchtige organische Material einschließlich Pentafluorbutan aus dem Teer zu verdampfen, der verdampfte HF und das flüchtige organische Material einschließlich Pentafluorbutan aus dem Teer mit dem Produktstrom vereinigt werden, das erhaltene Gemisch auf eine Temperatur von etwa −26,1 bis −9,44°C (−15 bis +15°F) abgekühlt wird, so daß das Gemisch in eine HF-reiche flüssige Phase und eine an 1-Chlor-1,1-difluorethan reiche flüssige Phase getrennt wird, und daß die zwei Phasen getrennt werden, wobei eine gute Temperaturkontrolle während der Trennung durchgeführt wird.

2. Verfahren nach Anspruch 1, dadurch gekennzeichnet, daß das Gemisch auf eine Temperatur von etwa −20,0°C (etwa −4°F) abgekühlt wird.

3. Verfahren nach Anspruch 1, dadurch gekennzeichnet, daß das nichtumgesetzte und teilweise umgesetztes organisches Material aus dem Produktstrom entfernt und die genannten Materialien in das Reaktionsgemisch zurückgeführt werden.

4. Verfahren nach Anspruch 1, dadurch gekennzeichnet, daß HCl aus dem Produktstrom entfernt wird.

5. Verfahren nach Ansprüche 1, dadurch gekennzeichnet, daß die HF-reiche flüssige obere Phase in das Reaktionsgemisch zurückgeführt wird.

6. Verfahren nach Anspruch 1, dadurch gekennzeichnet, daß ein azeotropes Gemisch aus HF und 1-Chlor-1,1-difluorethan aus der an 1-Chlor-1,1-difluorethan reichen flüssigen Phase abdestilliert wird und das azeotrope Gemisch mit dem Produktstrom und dem HF und dem flüchtigen organischen Material aus dem Teer zur Kühlung und Trennung der Phasen vereinigt wird.

7. Verfahren nach Anspruch 1, dadurch gekennzeichnet, daß der HF und das flüchtige organische Material aus dem Teer abgekühlt werden, bevor sie dem Produktstrom zugemischt werden.

**Revendications**

1. Procédé pour séparer du fluorure d'hydrogène des constituants organiques d'un mélange réactionnel contenant du pentafluorobutane, consistant à prélever un courant de produit qui comprend du HF et du 1-chloro-1,1-difluoréthane du mélange réactionnel, à prélever un courant de goudron du mélange réactionnel, à chauffer le goudron pour vaporiser le HF et la matière organique volatile comprenant le pentafluorobutane venant du goudron, à réunir le HF vaporisé et la matière organique volatile comprenant le pentafluorobutane venant du goudron avec le courant de produit, à refroidir le mélange résultant à une température d'environ −26,1 à −9,44°C (−15 à +15°F) de manière que le mélange se sépare en une phase liquide riche en HF et en une phase liquide riche en 1-chloro-1,1-difluoréthane, et à séparer les deux phases, un bon réglage de température étant exercé au cours de la séparation.

2. Procédé suivant la revendication 1, dans lequel le mélange est refroidi à une température d'environ −20,0°C (environ −4°F).

3. Procédé suivant la revendication 1, comprenant l'élimination de la matière organique n'ayant pas réagi ou ayant partiellement réagi du courant de produit et à renvoyer lesdites matières au mélange réactionnel.

4. Procédé suivant la revendication 1, consistant à éliminer du HCl du courant de produit.

5. Procédé suivant la revendication 1, comprenant le recyclage de la phase supérieure liquide riche en HF dans le mélange réactionnel.

6. Procédé suivant la revendication 1, consistant à distiller un mélange azéotrope de HF et de 1-chloro-1,1-difluoréthane de la phase liquide riche en 1-chloro-1,1-difluoréthane et à réunir le mélange azéotrope avec le courant de produit et le HF et la matière organique volatile venant du goudron en vue du refroidissement et de la séparation des phases.

7. Procédé suivant la revendication 1, consistant à refroidir le HF et la matière organique volatile venant du goudron avant leur mélange avec le courant de produit.